# EUROPEAN PATENT SPECIFICATION

(11) **EP 2 231 071 B1**
(45) Date of publication and mention of the grant of the patent: **24.10.2012**
(21) Application number: 08863768.1
(22) Date of filing: 18.12.2008
(51) Int. Cl.: A61F 2/34, A61B 17/80

(54) **ACETABULAR PROSTHESIS**
HÜFTPFANNEN-PROTHESE
PROTHÈSE ACÉTABULAIRE

(30) Priority: 19.12.2007 IT BO20070832
(43) Date of publication of application: 29.09.2010
(73) Proprietor: Istituto Ortopedico Rizzoli, 40136 Bologna (IT)
(72) Inventor: DALLARI, Dante, I-40059 Medicina (IT); PIGNATTI, Giovanni, I-40137 Bologna (IT)
(74) Representative: Lanzoni, Luciano
(86) International application number: PCT/IB2008/055419
(87) International publication number: WO 2009/081346

(56) References cited:
- EP-A- 0 121 002
- EP-A- 0 265 712
- EP-A- 0 807 420
- WO-A-98/15240
- US-A- 5 980 574
- US-A1- 2001 047 174
- US-A1- 2002 016 595

## Description

### Technical Field

This invention refers to an acetabular prosthesis and, in particular to an acetabular prosthesis designed to be implanted in redo operations and/or in patients affected by degenerative or congenital diseases and/or severe morphological alterations of the acetabulum.

### Background Art

Surgical intervention is known to resolve problems connected with the hip joint.

Two bones are connected at the hip joint, the pelvis and the femur, the head of the femur being lodged inside an approximately hemispherical cavity known as the acetabulum.

Many of these surgical operations involve the application of prostheses, on one or both the components of the joint.

### Disclosure of the Invention

This invention refers exclusively to the portion of the joint prosthesis which is positioned in the pelvis and known as a cotyloid, acetabular prosthesis or acetabular cup.

The acetabular prosthesis is therefore normally implanted in the pelvis to resolve deficits of the hip joint attributable to the acetabulum.

The prosthesis is fixed in the acetabulum in the pelvis by means of various techniques, such as the application of screws, surgical cement and mechanical forcing or screwing, as for example described in documents US 5980574 and EP 0265712, the first disclosing the features of the preamble of claim 1.

Often, especially in so-called redo operations, that is to say when it is necessary to replace a previously implanted prosthesis, and/or in patients affected by degenerative bone tissue diseases, the surgeon is faced with significant difficulties related to the effective fixing of the new prosthesis to the pelvis.

In the first case, in effect, the fact that a prosthesis had already been implanted means that the walls of the acetabulum have been mostly removed or reabsorbed, thus reducing the areas of bone that can be used for effective anchoring; in the second case, on the other hand, these diseases means that it is not easy to identify a mechanically valid area close to the acetabulum in which the prosthesis can be firmly anchored.

In this context, the aim of the invention is to provide an acetabular prosthesis designed to be firmly anchored also in redo operations or in cases of degenerative or congenital diseases of the pelvic bone.

In particular, the aim of this invention is to provide an acetabular prosthesis which can be effectively and firmly implanted.

The technical characteristics of this invention, according to the aforesaid aims, can be clearly deduced from the content of the claims described below, in particular from claim 1 and, preferably, from any one of the dependent claims, directly or indirectly, from claim 1.

### Brief Description of the Drawings

The advantages of this invention will also become more evident from the detailed description which follows and which refers to the accompanying drawings which represent, a non-binding example and a preferred embodiment of the invention, in which:
- figure 1 shows a schematic side view of an example for an acetabular prosthesis not according to the invention;
- figure 2 shows a schematic view from above of the acetabular prosthesis shown in figure 1;
- figure 3 is a cross-section view along the line III-III indicated in figure 2;
- figures 4 and 5 show respective cross-section views of the acetabular prosthesis according to this invention in different implant steps;
- figure 6 shows a schematic side view of an acetabular prosthesis not according to the invention in the implanted configuration;
- figure 7 shows a schematic front view of the acetabular prosthesis shown in figure 6;
- figure 8 is a cross-section view along the line VIII-VIII indicated in figure 7.
- figure 9 shows a prospective view of a part of the prosthesis shown in figures 5 and 8;
- figure 10 shows a schematic side view of a part of the prosthesis shown in figures 4 to 8;
- figure 11 shows a schematic side view of an embodiment of the invention, incorporating features of the prosthesis shown in the foregoing figures.

### Description of the Preferred Embodiments of the Invention

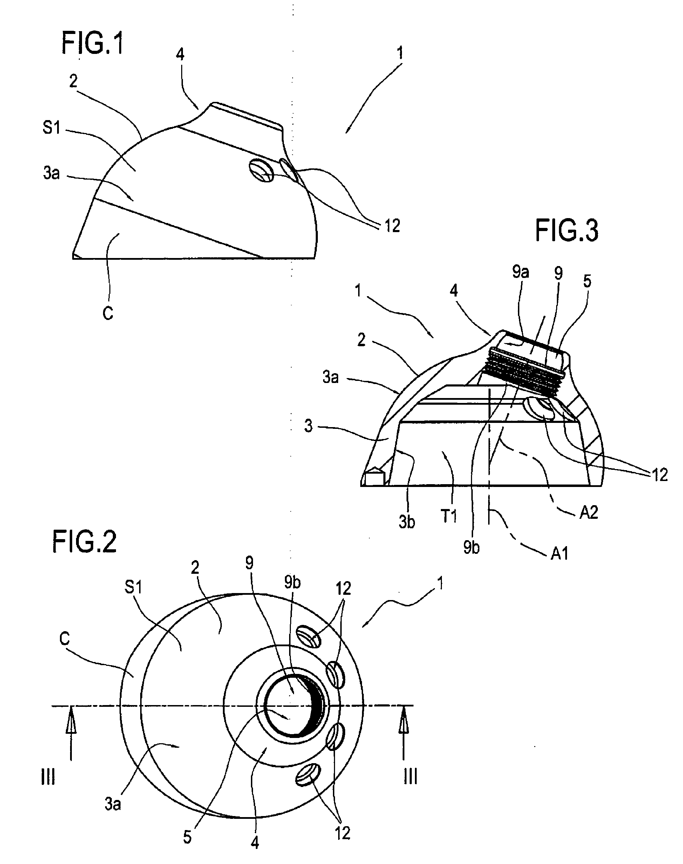

According to what is shown in figure 1, the reference number 1 indicates an acetabular prosthesis not falling under this invention.

The acetabular prosthesis 1 comprises an outer cup 2 designed to be inserted in a respective cavity, not show, formed starting from the natural acetabulum present in the patient's body, as will be better explained below.

The cup 2 comprises a wall 3 of varying thickness, this wall 3 presenting an outer face 3a substantially defined by a semispherical surface S1 and by a dissected cylindrical surface C with a plane which is inclined with respect to its central axis; the outer face 3a is substantially smooth.

The wall 3 also has an inner face 3b which is partially defined by a truncated cone shaped surface T1.

An approximately truncated cone shaped protrusion 4 extends from the wall 3, this protrusion being connected to the smooth outer face 3a of the cup 2.

The protrusion 4 is provided with a first through hole 5.

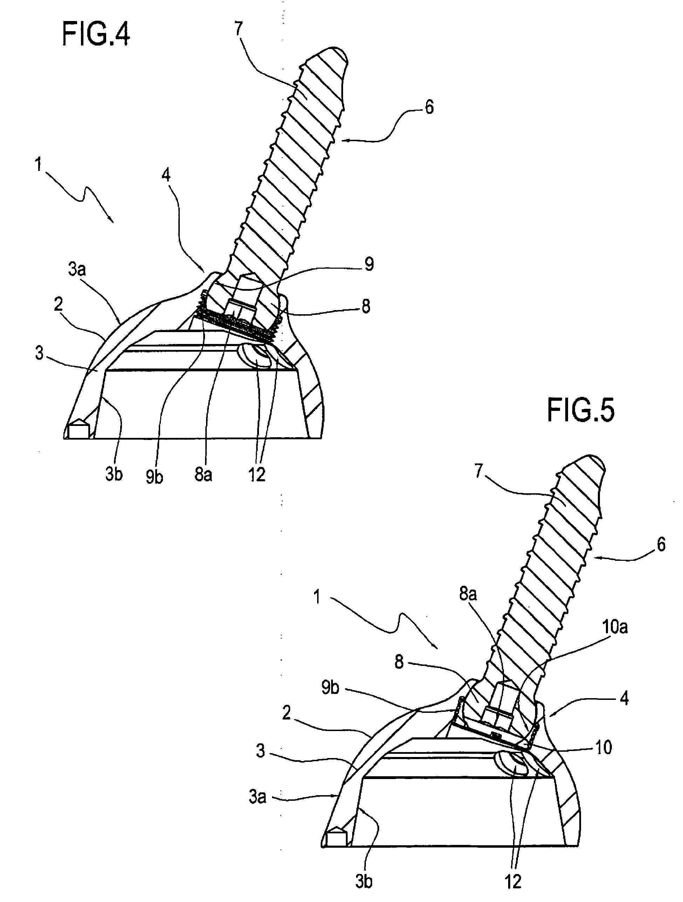

According to what is shown in figure 4, the acetabular prosthesis 1 comprises a first screw 6 for fixing the cup 2 to the pelvic bone.

The first screw 6 comprises a threaded shank 7 and a head 8.

The head 8 of the first screw 6 has a spherical area shape, meaning a solid geometrical shape obtained by cutting a sphere with two flat surfaces parallel to each other.

Inside the head 8 is a cavity 8a, advantageously hexagonal, designed to engage with appropriate means for screwing in of the first screw 6.

The first through hole 5 in the protrusion 4 is designed for the first screw 6 to pass through.

With reference to figure 3, the protrusion 4 also presents a concave housing 9, around the first hole 5, designed to restrain the head 8 of the first screw 6.

Advantageously, but not compulsorily, the concave housing 9 is counter-shaped with respect to the head 8 of the first screw 6, thus also presenting an inner surface 9a with a spherical area shape.

This defines a spherical joint for the first screw 6 between the head 8 and its respective housing 9.

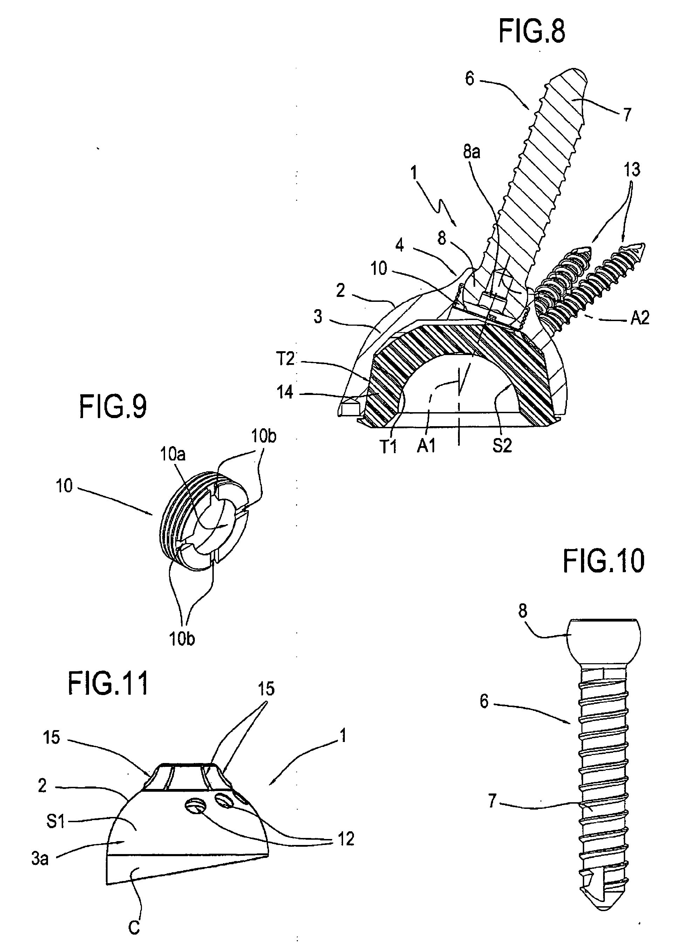

According to what is shown in figures 5 and 8, the acetabular prosthesis 1 comprises a threaded element 10 designed to engage in a respective threaded portion 9b of the housing 9.

According to what is shown in figure 9, the threaded element 10, which is internally hollow, presents a partially spherical inner surface 10a, meaning that this surface has a substantially spherical shape, similar to the surface 9a of the concave housing 9. The threaded element 10 also presents four notches 10b arranged in a cross and designed to engage appropriate means for the screwing in of the element 10.

As clearly shown in figure 5, the inner surface 10a of the threaded element 10 is designed to engage in contact with the head 8 of the first screw 6 in order to push the head 8 against the housing 9 and block it with respect to the cup 2 of which the housing 9 is an integral part.

The threaded element 10 thus defines means 11 for fixing the head 8 in the housing 9, designed to prevent relative movements between the first screw 6 and the cup 2 and vice versa.

According to what is shown in figures 2 and 3, the wall 3 of the cup 2 presents a plurality of second through holes 12, positioned at a distance from the first hole 5.

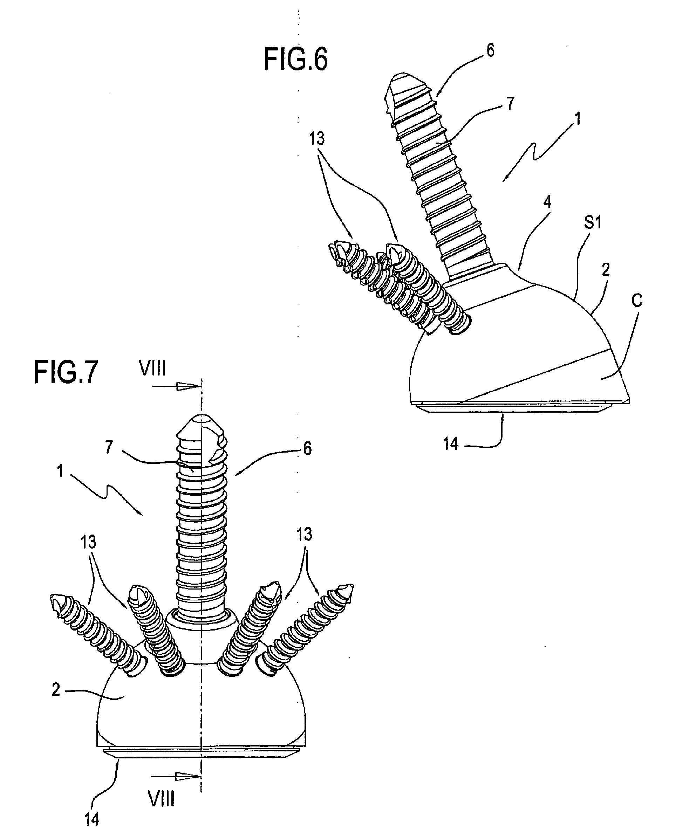

According to what is shown in figures 6 to 8, the acetabular prosthesis 1 also comprises a plurality of second screws 13 designed to be inserted in the second holes 12 and to engage against the ilium.

The inner surface of the second holes 12 has a partially spherical shape, as does the head, not shown, of the second screws 13 which, when inserted in the respective second holes 12, can therefore be positioned albeit with limited spherical movements around a respective centre common to the two counter-shaped spherical areas.

Advantageously, the first screw 6 is between 6 and 14 millimetres in diameter and between 40 and 120 millimetres long.

Advantageously, the second screws 13 are approximately 6 millimetres in diameter and between 20 and 60 millimetres long.

According to what is shown in figures 6, 7 and 8, the acetabular prosthesis 1 comprises an insert 14 with an outer truncated cone shaped surface T2, counter-shaped with respect to the inner truncated cone shaped surface T1 of'the wall 3 of the cup 2, and designed to engage in contact with it.

The insert 14 also comprises a substantially semispherical inner surface S2, designed to house the respective prosthesis head, not shown, of a femur.

The inner truncated cone shaped surface T1 of the wall 3 of the cup 2 presents a respective central axis A1 which is at an angle to the central axis A2 of the protrusion 4.

Advantageously, according to the example shown in the accompanying drawings, the axes A1, A2 are at an angle to each other.

Implantation of the acetabular prosthesis 1 according to this invention involves preparation of the patient's acetabulum, by means of appropriate cutters, in order to present a substantially semispherical shaped cavity with a radius equal to that of the semispherical surface S1 of the outer face 3a of the wall 3 of the cup 2.

The surgeon must therefore select the most appropriate area of the pelvic bone surrounding the cavity in which to house the first screw 6, this being the area providing the best guarantee of stable anchorage for the first screw 6.

A housing is then made, using an appropriate cutter if necessary, in the wall of the cavity for the protrusion 4 of the cup 2.

The cup 2 is then inserted in the cavity and the first screw 6 is screwed into the pelvic bone, having been passed through the first hole 5 in the protrusion 4.

The first screw 6 is screwed in until the head 8 is in contact with the housing 9 in the protrusion 4.

With the respective spherically shaped surfaces of the head 8 and the housing 9 in contact with each other, the threaded element 10 is screwed into the respective threaded portion 9b of the housing 9 until it blocks the head 8 against its spherical surface 9a, establishing the reciprocal fixing of the first screw 6 and the cup 2.

Exploiting the albeit limited positioning possibility of the second screws 13 within the respective second holes 12, the second screws 13 are screwed into the pelvic bone around the cavity, thus contributing further to the stabilization of the cup 2 in the patient's pelvis.

An important advantage of the acetabular prosthesis according to this invention consists of the substantial independence between the first screw 6 and the cup 2 in their respective positioning; thanks in fact to this distinctive feature, the surgeon can arbitrarily choose the position of the axis of the first screw even after positioning the cup 2 inside the patient's acetabulum.

According to an embodiment of the present invention shown in figure 11, the acetabular prosthesis 1 comprises a plurality of ribs 15 distributed around the outer surface of the protrusion 4 and developing longitudinally along the axis A2 of the protrusion 4. the ribs 15 contribute advantageously to holding the acetabular prosthesis 1 firmly in position in its housing, making it more difficult for the acetabular prosthesis 1 to rotate around the axis A2 of the protrusion 4.

In other words, thanks to the ribs 15, it is difficult for the protrusion 4 to rotate within the cavity in which it is housed because the ribs 15 engage firmly with the walls of the cavity.
Advantageously, the acetabular prosthesis 1 and the screws 6, 13 are made from titanium alloy or made from and/or coated with osteoinductive and/or osteoconductive materials, biofilm with the possibility of pharmacological and/or growth factor release

## Claims

1. An acetabular prosthesis comprising a cup (2) designed to be inserted in a relative acetabulum and a first screw (6) for fixing the cup (2) designed to engage by screwing in a portion of pelvic bone, said cup (2) comprising a wall (3) with a substantially smooth outer face (3a), and a protrusion (4) extending outwards from the wall (3) and connected to the smooth face (3a); said protrusion (4) presenting a first through hole (5) for the insertion of said first screw (6), and a housing (9) to restrain a respective head (8) of the screw (6) **characterised in that** the acetabular prosthesis further comprises a plurality of ribs (15) distributed around an outer surface of the protrusion (4) and developing longitudinally along a central axis (A2) of the protrusion (4).

2. An acetabular prosthesis according to claim 1, **characterised in that** the head (8) of the first screw (6) has a spherically shaped area.

3. An acetabular prosthesis according to claim 1 or 2, **characterised in that** the housing (9) is at least partially concave and counter-shaped with respect to said head (8) to define a spherical joint for said first screw (6).

4. An acetabular prosthesis according to claim 3, **characterised in that** it comprises means (11) for fixing said head (8) in said housing (9), to prevent relative movements between said first screw (6) and said cup (2).

5. An acetabular prosthesis according to claim 4, **characterised in that** said fixing means (11) comprise a threaded element (10) which engages by screwing with a respective threaded portion (9b) of said housing (9).

6. An acetabular prosthesis according to claim 5, **characterised in that** said threaded element (10) presents a partially spherical surface (10a) designed to engage in contact with said head (8) of said first screw (6).

7. An acetabular prosthesis according to any of the foregoing claims from 1 to 6, **characterised in that** it comprises at least one second screw (13) for fixing the cup (2), said second screw (13) being inserted in a respective second through hole (12) in the wall (3) of the cup (2) at a distance from said protrusion (4) and being designed to engage against the ilium.

8. An acetabular prosthesis according to claim 7, **characterised in that** said second screw (13) comprises a respective head with a spherically shaped area.

9. An acetabular prosthesis according to claim 7 or 8, **characterised in that** it comprises a plurality of said second screws (13).

10. An acetabular prosthesis according to any of the foregoing claims from 1 to 9, **characterised in that** said first screw (6) is between 6 and 14 millimetres in diameter.

11. An acetabular prosthesis according to any of the foregoing claims from 1 to 10, **characterised in that** said first screw (6) is between 40 and 120 millimetres long.

12. An acetabular prosthesis according to any of the foregoing claims from 7 to 11, **characterised in that** said second screw (13) is approximately 6 millimetres in diameter.

13. An acetabular prosthesis according to any of the foregoing claims from 1 12, **characterised in that** said wall (3) of the cup (2) presents an inner truncated cone shaped surface (T1) with a respective central axis (A1) separate from the central axis (A2) of said protrusion (4).

14. An acetabular prosthesis according to claim 13, **characterised in that** said central axes (A1, A2) are at an angle to each other.

15. An acetabular prosthesis according to claim 13 or 14, **characterised in that** it comprises an insert (14) presenting an outer truncated cone shaped surface (T2), counter-shaped with respect to the inner surface (T1) of the wall (3) of the cup (2) and designed to engage in contact with it, and a substantially semispherical inner surface (S2).

## Patentansprüche

1. Hüftpfannenprothese, umfassend eine Pfanne (2), die dazu konzipiert ist, in eine entsprechende Hüftpfanne eingeführt zu werden und eine erste Schraube (6) zum Fixieren der Pfanne (2), die zum Eingriff durch Schrauben in einen Beckenknochenabschnitt konzipiert ist, wobei die Pfanne (2) eine Wand (3) mit einer im Wesentlichen glatten Außenfläche (3a) und einen Vorsprung (4) umfasst, der sich von der Wand (3) nach Außen erstreckt und mit der glatten Fläche (3a) verbunden ist; wobei der Vorsprung (4) eine erste Durchgangsbohrung (5) für das Einführen der ersten Schraube (6) und ein Gehäuse (9) zum Festhalten eines entsprechenden Kopfes (8) der Schraube (6) aufweist, **dadurch gekennzeichnet, dass** die Hüftpfannenprothese ferner eine Vielzahl von Rippen (15) umfasst, die um eine Außenfläche des Vorsprungs (4) herum verteilt sind und sich longitudinal entlang einer Mittelachse (A2) des Vorsprungs (4) entwickeln.

2. Hüftpfannenprothese nach Anspruch 1, **dadurch gekennzeichnet, dass** der Kopf (8) der ersten Schraube (6) einen kugelförmigen Bereich aufweist.

3. Hüftpfannenprothese nach Anspruch 1 oder 2, **dadurch gekennzeichnet, dass** das Gehäuse (9) zumindest teilweise konkav und gegensätzlich zum Kopf (8) geformt ist, um ein Kugelgelenk für die erste Schraube (6) zu definieren.

4. Hüftpfannenprothese nach Anspruch 3, **dadurch gekennzeichnet, dass** sie Mittel (11) zum Fixieren des Kopfes (8) im Gehäuse (9) umfasst, um relative Bewegungen zwischen der ersten Schraube (6) und der Pfanne (2) zu verhindern.

5. Hüftpfannenprothese nach Anspruch 4, **dadurch gekennzeichnet, dass** die Fixiermittel (11) ein Gewindeelement (10) umfassen, das durch Schrauben in einen entsprechenden Gewindeabschnitt (9b) des Gehäuses (9) eingreift.

6. Hüftpfannenprothese nach Anspruch 5, **dadurch gekennzeichnet, dass** das Gewindeelement (10) eine teilweise kugelige Oberfläche (10a) aufweist, die dazu konzipiert ist, am Kopf (8) der ersten Schraube (6) anliegend einzugreifen.

7. Hüftpfannenprothese nach einem der vorangehenden Ansprüche 1 bis 6, **dadurch gekennzeichnet, dass** sie mindestens eine zweite Schraube (13) zum Fixieren der Pfanne (2) umfasst, wobei die zweite Schraube (13) in eine entsprechende zweite Durchgangsbohrung (12) in der Wand (3) der Pfanne (2) vom Vorsprung (4) beabstandet eingeführt und zum Eingriff am Darmbein konzipiert ist.

8. Hüftpfannenprothese nach Anspruch 7, **dadurch gekennzeichnet, dass** die zweite Schraube (13) einen entsprechenden Kopf mit einem kugelförmigen Bereich aufweist.

9. Hüftpfannenprothese nach Anspruch 7 oder 8, **dadurch gekennzeichnet, dass** sie eine Vielzahl der zweiten Schrauben (13) umfasst.

10. Hüftpfannenprothese nach einem der vorangehenden Ansprüche 1 bis 9, **dadurch gekennzeichnet, dass** die erste Schraube (6) einen Durchmesser von 6 bis 14 Millimetern aufweist.

11. Hüftpfannenprothese nach einem der vorangehenden Ansprüche 1 bis 10, **dadurch gekennzeichnet, dass** die erste Schraube (6) eine Länge von 40 bis 120 Millimetern aufweist.

12. Hüftpfannenprothese nach einem der vorangehenden Ansprüche 7 bis 11, **dadurch gekennzeichnet, dass** die zweite Schraube (13) einen Durchmesser von ca. 6 Millimetern aufweist.

13. Hüftpfannenprothese nach einem der vorangehenden Ansprüche 1 bis 12, **dadurch gekennzeichnet, dass** die Wand (3) der Pfanne (2) eine kegelstumpfförmige Innenfläche (T1) mit einer entsprechenden Mittelachse (A1) aufweist, die von der Mittelachse (A2) des Vorsprungs (4) verschieden ist.

14. Hüftpfannenprothese nach Anspruch 13, **dadurch gekennzeichnet, dass** die Mittelachsen (A1, A2) winklig zueinander sind.

15. Hüftpfannenprothese nach Anspruch 13 oder 14, **dadurch gekennzeichnet, dass** sie ein Inlay (14) umfasst, das eine kegelstumpfförmige Außenfläche (T2), die gegensätzlich zur Innenfläche (T1) der Wand (3) der Pfanne (2) geformt und dazu konzipiert ist, daran anliegend einzugreifen, sowie eine halbkugelförmige Innenfläche (S2) aufweist.

## Revendications

1. Prothèse acétabulaire comprenant une cupule (2) conçue pour être insérée dans une cavité cotyloïdienne et une première vis (6) pour fixer la cupule (2) conçue pour s'engager par vissage dans une portion de l'os iliaque, ladite cupule (2) comprenant une paroi (3) avec une face externe essentiellement lisse (3a), et une saillie (4) s'étendant à l'extérieur de la paroi (3) et reliée à la face lisse (3a) ; ladite saillie (4) présentant un premier trou passant (5) pour l'insertion de ladite première vis (6), et un logement (9) pour retenir une tête correspondante (8) de la vis (6) **caractérisée en ce que** la prothèse acétabulaire comprend également une pluralité de nervures (15) distribuées autour d'une surface externe de la saillie (4) et se développant longitudinalement le long d'un axe central (A2) de la saillie (4).

2. Prothèse acétabulaire selon la revendication 1, **caractérisée en ce que** la tête (8) de la première vis (6) présente une zone ayant une forme sphérique.

3. Prothèse acétabulaire selon la revendication 1 ou 2, **caractérisée en ce que** le logement (9) est au moins partiellement concave et ayant une forme complémentaire par rapport à ladite tête (8) pour définir un joint sphérique pour ladite première vis (6).

4. Prothèse acétabulaire selon la revendication 3, **caractérisée en ce qu'**elle comprend un moyen (11) pour fixer ladite tête (8) dans ledit logement (9), pour prévenir les mouvements correspondants entre ladite première vis (6) et ladite cupule (2).

5. Prothèse acétabulaire selon la revendication 4, **caractérisée en ce que** ledit moyen de fixation (11) comprend un élément fileté (10) qui s'engage par vissage dans une portion filetée respective (9b) du dit logement (9).

6. Prothèse acétabulaire selon la revendication 5, **caractérisée en ce que** ledit élément fileté (10) présente une surface partiellement sphérique (10a) conçue pour s'engager en contact de ladite tête (8) de ladite première vis (6).

7. Prothèse acétabulaire selon l'une quelconque des revendications 1 à 6, **caractérisée en ce qu'**elle comprend au moins une deuxième vis (13) pour fixer la cupule (2), ladite deuxième vis (13) étant insérée dans un second trou passant respectif (12) dans la paroi (3) de la cupule (2) à une distance de ladite saillie (4) et étant conçue pour s'engager contre l'os iliaque.

8. Prothèse acétabulaire selon la revendication 7, **caractérisée en ce que** ladite deuxième vis (13) comprend une tête respective avec une zone ayant une forme sphérique.

9. Prothèse acétabulaire selon la revendication 7 ou 8, **caractérisée en ce qu'**elle comprend une pluralité desdites deuxièmes vis (13).

10. Prothèse acétabulaire selon l'une quelconque des revendications précédentes 1 à 9, **caractérisée en ce que** ladite première vis (6) a un diamètre de 6 à 14 millimètres.

11. Prothèse acétabulaire selon l'une quelconque des revendications précédentes 1 à 10, **caractérisée en ce que** ladite première vis (6) a une longueur de 40 à 120 millimètres.

12. Prothèse acétabulaire selon l'une quelconque des revendications précédentes 7 à 11, **caractérisée en ce que** ladite deuxième vis (13) a un diamètre approximatif de 6 millimètres.

13. Prothèse acétabulaire selon l'une quelconque des revendications précédentes 1 à 12, **caractérisée en ce que** ladite paroi (3) de la cupule (2) présente une surface interne en forme de cône tronconique (T1) avec un axe central respectif (A1) séparé de l'axe central (A2) de ladite saillie (4).

14. Prothèse acétabulaire selon la revendication 13, **caractérisée en ce que** lesdits axes centraux (A1, A2) se trouvent à un angle par rapport à l'autre.

15. Prothèse acétabulaire selon la revendication 13 ou 14, **caractérisée en ce qu'**elle comprend un insert (14) présentant une surface externe en forme de cône tronconique (T2), contre-profilé par rapport à la surface interne (T1) de la paroi (3) de la cupule (2) et conçue pour s'engager en contact avec cette dernière, et une surface (S2) interne essentiellement hémisphérique.
